# EUROPÄISCHE PATENTSCHRIFT

(11) **EP 0 975 426 B1**
(45) Veröffentlichungstag und Bekanntmachung des Hinweises auf die Patenterteilung: **15.01.2003**
(21) Anmeldenummer: 98913749.2
(22) Anmeldetag: 02.04.1998
(51) Int. Cl.: B01J 31/02, B01J 31/12, C07D 301/00, C07F 7/28

(54) **VERFAHREN ZUR HERSTELLUNG VON SILASESQUIOXAN-METALLKOMPLEXEN, NEUE SILASESQUIOXAN-METALLKOMPLEXE UND DEREN VERWENDUNG**
METHOD FOR PRODUCING SILASESQUIOXANE METAL COMPLEXES, NOVEL SILASESQUIOXANE METAL COMPLEXES AND THE USE THEREOF
PROCEDE POUR LA PREPARATION DE COMPLEXES METALLIQUES DE SILASESQUIOXANE, NOUVEAUX COMPLEXES METALLIQUES DE SILASESQUIOXANE ET LEUR UTILISATION

(30) Priorität: 16.04.1997 DE 19715786; 05.11.1997 DE 19748835
(43) Veröffentlichungstag der Anmeldung: 02.02.2000
(73) Patentinhaber: Creavis Gesellschaft für Technologie und Innovation mbH, 45764 Marl (DE)
(72) Erfinder: VAN SANTEN, Rutger, Anthony, NL-1817 HX Alkmaar (NL); ABBENHUIS, Hendrikus, Cornelis, Louis, NL-4811 GK Breda (NL); VORSTENBOSCH, Martinus, Lambertus, Wilhelmus, NL-5730 BT Mariahout (NL)
(86) Internationale Anmeldenummer: EP9801932
(87) Internationale Veröffentlichungsnummer: WO98046352

(56) Entgegenhaltungen:
- WO-A-97/24344
- I. E. BUYS ET AL.: "Models of surface-confined metallocene derivatives" JOURNAL OF MOLECULAR CATALYSIS, Bd. 86, 1994, AMSTERDAM, Seiten 309-318, XP002069593
- F. FEHER ET AL.: "Silasesquioxanes as ligands in inorganic and organometallic chemistry" POLYHEDRON, Bd. 14, Nr. 22, 1995, Seiten 3239-3253, XP002069936 in der Anmeldung erwähnt
- N. WINKHOFER ET AL.: "Stable Silanetriols as building blocks for the Synthesis of Titanasilsesquioxanes-Model Compounds for Ti-doped Zeolites" ANGEW. CHEM. IND. ENG. ENGL., COMMUNICATIONS, Bd. 33, Nr. 13, 1994, WEINHEIM, Seiten 1352-1354, XP002028743

## Beschreibung

Die Erfindung betrifft ein Verfahren zur Herstellung von Silasesquioxan-Metallkomplexen, neue Silasesquioxan-Metallkomplexe sowie deren Verwendung.

Die Herstellung von Silasesquioxan-Metallkomplexen durch Umsetzung von Trisilanol, das mit Cyclohexylgruppen substituiert ist, mit einer Metallverbindung ist bekannt. (Polyhedron, 1995, Vol. 14, Nr. 22, S. 3239 - 3253)

Die Herstellung von Sisasesquioxan-Metallkomplexen mit Cyclopentadienylliganden ist ebenfalls bekannt. (Journal of Molecular catalysis, Bd. 86, 1994, S. 309 - 318).

In beiden Fällen werden Silasesquioxane mit mindestens 7 Si-Atomen im Cluster eingesetzt.

Die Herstellung eines Cyclohexylgruppen-haltigen Trisilanols ist sehr zeitaufwendig. Dieses Trisilanol ist jedoch sehr gut löslich, so daß die Umsetzung zu den gewünschten Metallkomplexen ohne weiteres möglich ist.

Die Herstellung eines Trisilanols, das mit Cyclopentylgruppen substituiert ist, ist in sehr viel kürzerer Zeit möglich. Das Trisilanol ist jedoch schlecht löslich, so daß die Herstellung der entsprechenden Metallkomplexe bisher nicht beschrieben wurde.

Die bisher bekannten Verfahren zur Herstellung der Metallkomplexe sind sehr zeitaufwendig und benötigen große Mengen an Lösungsmittel, die wiederum aufbereitet werden müssen.

Es ist ebenfalls bekannt, Titan-haltige Metallkomplexe als Katalysatoren bei der Herstellung von Epoxid-Verbindungen durch Oxidation ungesättigter Kohlenwasserstoffe zu verwenden. Beispielsweise wird in der WO 97/24344 ein Verfahren zur Oxidation von olefinisch ungesättigten Kohlenwasserstoffen offenbart, wobei Titan-Silasesquioxan-Katalysatoren verwendet werden. Jedoch ist dieses Verfahren auf organische Hydroperoxide als Oxidationsmittel beschränkt und kann nur in wasserfreien organischen Lösungsmitteln durchgeführt werden. Die Titanatome in den verwendeten Titan-Silasesquioxan-Katalysatoren sind 4-fach tetraedrisch koordiniert.

Die Aufgabe der Erfindung besteht darin, ein Verfahren zur Herstellung von Silasesquioxan-Metallkomplexen bereitzustellen, mit dem auch die bisher nicht oder nur schwer zugänglichen Silasesquioxan-Metallkomplexen effizient zur Verfügung gestellt werden können.

Die Aufgabe der Erfindung besteht weiterhin darin, die Silasesquioxan-Metallkomplexe als Katalysatoren für die Oxidation ungesättigter Kohlenwasserstoffe oder Alkohole zur Verfügung zu stellen, welche auch in wäßrigem Medium stabil und katalytisch aktiv sind, da auf diese Weise umweltfreundliche Oxidationsmittel wie Sauerstoff oder Wasserstoffperoxid beispielsweise für die Epoxidation von Alken-Verbindungen eingesetzt werden können.

Die Aufgabe der Erfindung wird dadurch gelöst, daß die Umsetzung der Metallverbindung mit einem Silasesquioxan mit definierter Zähnigkeit in einer Suspension erfolgt.

Erfindungsgemäß werden Halogenide, insbesondere Chloride, C₁-C₂₀-alkyl- oder Aryl-Verbindungen, die kein Betawasserstoffatom enthalten, z. B. Methyl-, Benzyl-, Neopentyl-, Xylyl-, Mesityl-, Neophil-, Adamantyl-Verbindungen, Silyl-, Fluorenyl-, Indenyl-, Cyclopentadienyl-Verbindungen, wobei die einzelnen Liganden durch C₁-C₄-alkyl-, C₁-C₄-alkylsilyl, alkoxy-, aryl- oder arylsilyl-Gruppen substituiert sein können,
Oxide, Imide, Amide, Alkoxide (z. B. -OR, wobei R Wasserstoff, C₁-C₂₀-alkyl, insbesondere methyl, ethyl, isopropyl, tert.-butyl, aryl, insbesondere benzyl, phenyl, toluyl, naphthyl, xylyl, bedeutet) oder deren Mischverbindungen, z. B. Oxohalogenide, Aryl- oder Alkylhalogenide, Halogenamide oder Alkylalkoxide der Metalle der 4. bis 7. Nebengruppe des PSE und mindestens ein Silasesquioxan der allgemeinen Formel I

{(R¹SiO_{1,5})ₙ(R²SiO_{1,5})ₘ[(H)ₚ(B)_{q}(O)ᵣ]}

in der
- R¹: C₅-C₁₀-cycloalkyl, insbesondere cyclopentyl, cyclohexyl, cycloheptyl, norbornyl, adamantyl
- R²: OH
- B: H, OH, halogen, alkoxy oder SiR³_{y}, wobei R³ C₁-C₄-alkyl, insbesondere methyl, aryl, insbesondere phenyl oder SiMe₂(CH₂)ₛCH = CH₂, SiMe₂ (CH₂)ₛCH₂CH₂A, SiMe₂(CH₂)ₛCHACH₃, wobei A für OH, COOH, NH₂, SO₃⁻, COO⁻ und s für 1 bis 20 steht, sein kann, y für 2 und 3 steht und
- R¹ und R³: durch Halogen oder OH funktionalisiert sein können
und
- n: 6
- m: 0 und 1
- p: 0 bis 4
- q: 0 bis 2
- r: 0 bis 2
bedeuten,
in einem organischen Lösungsmittel, z. B. einen alkylierten aromatischen Kohlenwasserstoff, gegebenenfalls in Gegenwart einer basischen Verbindung, unter Rühren bei -80 °C bis +110 °C suspendiert wird und das Reaktionsprodukt bei Raumtemperatur abgetrennt wird.

Als Metalle im Sinne der Erfindung werden insbesondere Titan, Zirkon, Hafnium, Chrom, Wolfram, Molybdän, Vanadium, Niob, Tantal, Rhenium, vorzugsweise Titan, Vanadium oder Zirkon verstanden.

Als Metallverbindung werden z. B. TiCl₄, TiCpCl₃, TiCl₃{η⁵-C₅H₃(SiMe₃)₂-1.3}, Zirkonalkylverbindung, Zirkonalkylarylverbindung, VO(Oi-alk)₃, insbesondere VO(Oi-prop)₃, VOCl₃ eingesetzt.

Als basische Verbindung wird vorzugsweise eine organische Base verwendet.

Durch einfaches Zentrifugieren werden die Nebenprodukte abgetrennt.

Aus der überstehenden Lösung können die Silasesquioxan-Metallkomplexe z. B. durch Zugabe von mit Wasser mischbaren protischen Lösungsmitteln wie i-Propanol, Ethanol, Aceton, Acetonitril direkt gefällt und in bekannter Weise gereinigt werden z. B. durch Umkristallisation.

Das erfindungsgemäße Verfahren zur Herstellung der Silasesquioxan-Metallkomplexe der allgemeinen Formel II

{(R¹SiO_{1,5})ₙ(R^{2a}SiO_{1,5})ₘ[(B)_{q}(O)ᵣ]}ᵤ(M)ᵥ(Y)_{w}

in der
R¹, B, n, m, q, r die obengenannte Bedeutung haben, R^{2a} Sauerstoff
- u: 1 bis 4
- v: 1 bis 4
- w: 0 bis 12
- M: für die Metalle und Y für die an das Metall gebundenen obengenannten Roste der Metallverbindung steht, bedeuten,
zeichnet sich gegenüber den bisher bekannten Verfahren dadurch aus, daß der Lösungsmittelbedarf drastisch gesenkt werden kann. Die zeitaufwendigen Aufarbeitungsstufen können somit entfallen.

In einer Ausführungsform werden als Metallverbindungen vorzugsweise Verbindungen von Titan, Zirkon eingesetzt, insbesondere wird TiCl₄, TiCl₃(η⁵-C₅H₅) oder TiCl₃{η⁵-C₅H₃(SiMe₃)₂1,3} oder Zr(CH₂C₆H₅)₄ eingesetzt.

In einer anderen Ausführungsform werden vorzugsweise Silasesquioxane der allgemeinen Formel I in der
n = 6, m = q = 0, p = 4, r = 2
R¹ = c-C₇H₁₃, norbornyl
bedeuten, verwendet.

In einer weiteren Ausführungsform werden silasesquioxane der allgemeinen Formel I, in der R¹ für c-C₅H₉ steht, verwendet.

In einer anderen Ausführungsform werden Titan-Silasesquioxan-Komplexe hergestellt, indem eine Titanverbindung mit einem Silasesquioxan (I) in einem alkylierten aromatischen Kohlenwasserstoff, vorzugsweise Toluol bei 20 bis 50 °C suspendiert wird. Unter Zugabe einer basischen Verbindung, vorzugsweise Pyridin, wird die Suspension unter Beibehaltung einer Temperatur von ca. 50 °C weitergerührt. Die Mischung wird auf Raumtemperatur abgekühlt und die Nebenprodukte, vorzugsweise durch Zentrifugieren, abgetrennt.

Es wurde gefunden. daß neue, 4- oder 6-fach koordinierte Metallkomplexe herstellbar sind, die für die Oxidation ungesättigter Kohlenwasserstoffe und Alkohole in wäßrigem Medium geeignete Katalysatoren darstellen.

Die erfindungsgemäßen neuen Metallkomplexe umfassen insbesondere Titanatome enthaltende Metallkomplexe, worin jedes Titanatom 6-fach koordiniert ist. Dieses Strukturmerkmal der 6-fachen Koordination aller Titanatome stellt eine wichtige Voraussetzung für die Hydrolysebeständigkeit der erfindungsgemäßen Metallkomplexe und für deren katalytische Wirksamkeit in wäßrigem Medium dar. Üblicherweise enthält ein in erfindungsgemäßen Metallkomplexen enthaltenes Metallcluster 4 tetraedrisch angeordnete Titanatome, wobei jedes Titanatom in erster Koordinationssphäre von 6 Sauerstoffatomen oktaedrisch umgeben ist. Diese Sauerstoffatome können neben der Bindung zu dem Titanatom noch weitere Bindungen, vorzugsweise zu Wasserstoff-, Kohlenstoff- oder Siliciumatomen, ausbilden. Es wurde gefunden, daß mindestens 2 der Titanatome in dem oxidischen Metallcluster über eine Hydroxylgruppe miteinander verbrückt sind. In der Regel wird der in den Metallkomplexen enthaltene Metallcluster gemeinsam durch 4 Titanatome und 4 Hydroxylgruppen gebildet, wobei je 3 Titanatome über eine Hydroxylgruppe miteinander verbrückt sind. Meist bilden die Titanatome auf diese Weise mit den verbrückenden Hydroxylgruppen ein Kuboid ( = verzerrter Kubus) aus, worin jeweils 3 der 4 Titanatome die gleiche koordinative Umgebung besitzen und jeweils 3 der 4 Hydroxylgruppen dieselbe Umgebung besitzen. Auf diese Weise werden 3 der 6 Koordinationsstellen der Titanatome durch jeweils eine Bindung zu einer verbrückenden Hydroxylgruppe besetzt. In einer bevorzugten Ausführungsform werden die verbleibenden 3 Koordinationsstellen der Titanatome durch Sauerstoffatome, welche von mindestens 4-zähnigen Liganden stammen, besetzt. Vorteilhaft ist es, wenn die 3 Koordinationsstellen der vorgenannten Titanatome, welche keine Bindungen zu verbrückenden Hydroxylgruppen ausbilden, von zu Silasesquioxan-Liganden stammenden Sauerstoffatomen besetzt werden. Beispielsweise können diese nicht Hydroxyl-verbrückenden Koordinationsstellen der Titanatome durch insgesamt drei 4-zähnige Silasesquioxan-Liganden der allgemeinen Formel [R₆(Si₆O₇)O₄], worin R für C₅-C₁₀-Cycloalkyl, Adamantyl oder für Norbornyl steht, besetzt werden.

Besonders bevorzugte erfindungsgemäße Metallkomplexe stellen die Metallkomplexe der allgemeinen Formel III 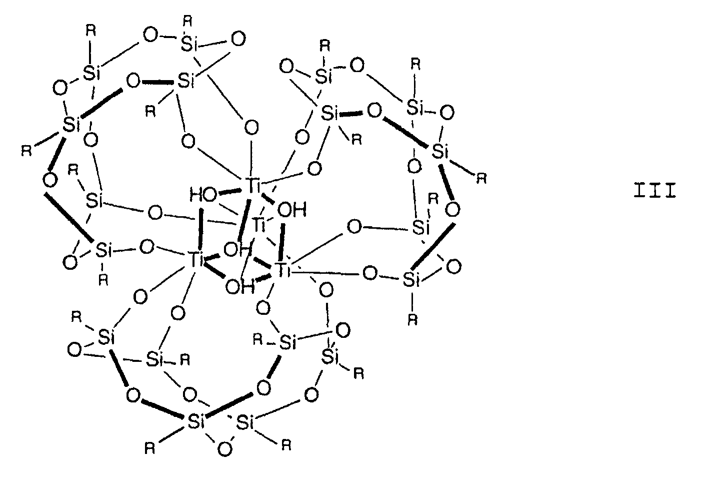 dar, worin R die obige Bedeutung für R¹ besitzt.

In diesen Verbindungen der Formel III steht R vorzugsweise für Cycloheptyl oder für Norbornyl.

Verbindungen der Formel III können hergestellt werden, indem man Verbindungen der allgemeinen Formel IV 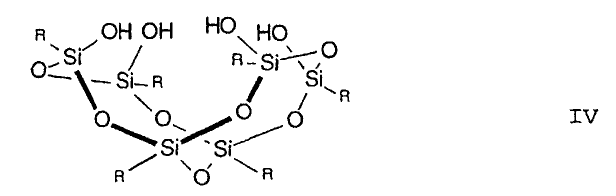 worin R obige Bedeutung für R¹ besitzt, mit Titantetrachlorid umsetzt.

Tetrasilanole der Formel IV sind zum Teil bekannt aus Feher et al. Organometallics 10 (1991), Seiten 2526 bis 2528 sowie Feher et al, Polyhedron 14 (1995), Seiten 3239 bis 3253 und können nach den dort beschriebenen Verfahren oder analog zu diesen Verfahren hergestellt werden.

In einer weiteren Ausführungsform erfolgt die Umsetzung der Tetrasilanole der Formel IV mit Titantetrachlorid bei Temperaturen von - 60 bis 110 °C. Vorzugsweise werden die Reaktanden bei Raumtemperatur vermischt und die Temperatur wird anschließend zur Vervollständigung der Reaktion für 1 bis 10 Minuten, vorzugsweise für 2 Minuten, auf 30 °C bis 80 °C, vorzugsweise auf 40 °C bis 60 °C, erhitzt und vor der Aufarbeitung wieder auf Raumtemperatur abgekühlt. Als Lösungsmittel können organische Lösungsmittel wie Methylenchlorid oder gegebenenfalls ein- oder mehrfach im Phenylring durch niederes Alkyl substituierte Benzole, beispielsweise Toluol, verwendet werden. Zweckmäßig kann dem Reaktionsgemisch vor dem Erhitzen eine geringe Menge einer organischen Base wie ein tertiäres Niederalkylamin, beispielsweise Triethylamin oder Pyridin, zugesetzt werden.

Die Metallkomplexe der Formel III werden bei der Herstellung als Racemate erhalten. Gegenstand der Erfindung sind daher sowohl die racemischen Gemische wie die reinen Enantiomere von Metallkomplexen der Formel III.

In einer anderen bevorzugten Ausführungsform wird eine Zirkonverbindung mit einem Silasesquioxan in Toluol bei -80 °C suspendiert. Die Mischung wird unter Rühren auf Raumtemperatur erwärmt und die Nebenprodukte in bekannter Weise abgetrennt.

Es wurde gefunden, daß die Herstellung dieser Zirkon-Silasesquioxan-Komplexe möglich ist, ohne daß eine basische Verbindung wie Pyridin zugesetzt werden muß.

Das erfindungsgemäße Verfahren ermöglicht beispielsweise die Herstellung von Silasesquioxan-Metallkomplexen der allgemeinen Formel II, in der
1)
   - n =: 6, m = q = 0, r = 2, u = 1, v = 1, w = 0
   - R¹ =: c-C₇H₁₃, norbornyl
   - M =: Ti, Zr, Hf
2)
   - n =: 6, m = q = 0, r = 2,
   - u =: v = w = 1
   - R¹ =: c-C₇H₁₃, norbornyl
   - M =: Cr, MO, W
   - Y =: oxo, imido
3)
   - n =: 6, m = q = 0, r = 2, u = 1, v = 2,
   - w =: 4
   - R¹ =: c-C₇H₁₃, norbornyl
   - M =: Cr, Mo, W
   - Y =: oxo, imido
4)
   - n =: 6, m = g = 0, r= 2, u = 1
   - v =: 4, w = 12
   - R¹ =: c-C₇H₁₃, norbornyl
   - M =: Re
   - Y =: oxo, imido
bedeuten.

Das erfindungsgemäße Verfahren ermöglicht die Herstellung von Silasesquioxan-Metallkomplexen, die unter Verwendung von Cyclopentylgruppen-baltigen Silasesquioxanen hergestellt wurden. Es wurde gefunden, daß diese Metallkomplexe in sehr kurzer Zeit herstellbar sind. Die Reaktionszeit beträgt nur wenige Minuten.

Derartige Metallkomplexe konnten bisher aufgrund der schlechten Löslichkeit des Cyclopentylgruppen-haltigen Silasesquioxans nicht hergestellt werden.

Das erfindungsgemäße Verfahren ermöglicht weiterhin die Herstellung von ein- und mehrzähnigen Silasesquioxan-Metallkomplexen. Es ist ebenfalls möglich, vierfach und sechsfach koordinierte Metallkomplexe herzustellen. Die Herstellung von Silasesquioxan-Polymetallkomplexen ist ebenfalls möglich.

Die erfindungsgemäß hergestellten Metallkomplexe sind stabile, mikrokristalline Feststoffe.

Es wurde weiterhin gefunden, daß die erfindungsgemäß hergestellten Metallkomplexe katalytisch wirksam sind und insbesondere als Katalysator für die Oxidation oder Epoxidierung von ungesättigten Kohlenwasserstoffen, einschließlich von halogenierten Alkenen und Alkoholen geeignet sind.

Die erfindungsgemäße Verwendung der Silasesquioxan-Metallkomplexe als Katalysator ist, da die aktiven Zentren im Metallkomplex genau definiert sind und somit das leaching-Verhalten kontrolliert und gesteuert werden kann, als besonders vorteilhaft gegenüber den bisher bekannten und verwendeten Katalysatoren anzusehen.

Insbesondere können die erhaltenen Metallkomplexe der Formel III als Katalysatoren für die Oxidation oder Epoxidation ungesättigter Kohlenwasserstoffe oder Alkohole verwendet werden. Hierzu können Verbindungen der Formel III gewünschtenfalls auf an sich bekannte Weise auf üblichen oxidischen Katalysatorträgern wie Siliciumdioxid, vorzugsweise auf Katalysatorträgern mit einer durchschnittlichen Porenweite zwischen 30 und 200 Å, aufgebracht werden. In durch die erfindungsgemäßen Metallkomplexe katalysierten Oxidationsreaktionen können organische oder anorganische Oxidationsmittel verwendet werden. Geeignete organische Oxidationsmittel sind beispielsweise organische Hydroperoxide wie tert.-Butylhydroperoxid (= TBHP) oder Ethylbenzolhydroperoxid. Geeignete anorganische Oxidationsmittel sind beispielsweise Wasserstoffperoxid. Die Oxidationsreaktionen können in geeigneten organischen Lösungsmitteln wie z. B. Alkohole, Hexan, Toluol oder in wäßrigem Medium durchgeführt werden.

Werden die Oxidationsreaktionen in wäßrigen Medien durchgeführt, ist die Verwendung von mehrzähnigen Silasesquioxan-Metallkomplexe erforderlich. Es wurde gefunden, daß die mehrzähnigen Metallkomplexe in wäßrigen Systemen gar nicht oder nur teilweise hydrolisieren. Damit behalten sie eine definierte Struktur und somit ihre katalytische Aktivität.

Die nachfolgenden Beispiele sollen die Erfindung erläutern, jedoch nicht einschränken.

### Test der katalytischen Wirksamkeit

Die Tests zur katalytischen Wirksamkeit der erfindungsgemäßen Metallkomplexe wurden in einem mit einem Rührwerk versehenen Batch-Reaktor von 1,5 ml Volumen und bei kontrollierter Temperatur durchgeführt. Für jeden Versuch wurden als Oxidationskatalysator jeweils 4,4 mg [(c-O₇H₁₃)₆Si₆O₁₁]₃[TiOH]₄ in 750 µl Toluol gelöst. Anschließend wurden 750 µl des entsprechenden Alkens zugegeben. Das entstandene Gemisch wurde jeweils auf 80 °C aufgeheizt, bevor eine Lösung des Oxidationsmittels in einem geeigneten organischen oder anorganischen Lösungsmittel zugegeben wurde. Die Menge des in den einzelnen Versuchen durch Oxidation der ungesättigten Ausgangsverbindungen erhaltenen epoxidierten Produktes wurde jeweils mittels ¹H-NMR-Spektroskopie bestimmt und in der nachfolgenden Tabelle als Prozentwert des Epoxids in Bezug auf die Ausgangsmenge an Peroxid angegeben.

**Tabelle**

| **Bsp. Nr.** | **Alken** | **Oxidans** | **T (°C)** | **Alken/Oxi- dans** | **Oxidans/Kata- lysator** | **Umwandlung des Oxidans (%)** | **Selektivität zum Epoxid (%)** |
|---|---|---|---|---|---|---|---|
| 1 | Cycloocten | TBHP | 80 | 23 | 178 | 100 | 60 (0,5 h) |
| 2 | 1-Octen | TBHP | 80 | 19 | 178 | 100 | 20 (1 h) 60 (18 h) |
| 3 | Cycloocten | H₂O₂ | 80 | 48,24,16,12 | 85, 171, 257, 343 | 100 | >90 (1 h) |
| 4 | 1-Octen | H₂O₂ | 80 | 40 | 85 | 100 | 10 (3 h) |

In den Versuchen 1 und 2 wurde eine Lösung von TBHP in Toluol als Oxidationsmittel verwendet. Die Gesamtmenge an TBHP betrug jeweils 22,5 mg. In den Versuchen 3 und 4 wurde eine wäßrige Lösung von Wasserstoffperoxid als Oxidationsmittel verwendet. In diesen Fällen wurde nur die halbe Menge an Oxidationsmittel verwendet (10 µl einer 35%igen wäßrigen Wasserstoffperoxid-Lösung) und das Reaktionsgemisch wurde nach einer Stunde auf vollständigen Umsatz kontrolliert. In Versuch 3 wurde die Reaktion anschließend durch Zugabe von weiterem Wasserstoffperoxid erneut gestartet und das Reaktionsgemisch nach Ablauf einer weiteren Stunde erneut auf seinen Gehalt an epoxidiertem Produkt kontrolliert. Dieser Vorgang konnte dreimal wiederholt werden, ohne daß eine Abnahme der katalytischen Aktivität des Titan-Komplexes beobachtet wurde. Dies belegt, daß die katalytische Verbindung gegen Hydrolyse und H₂O₂ stabil ist.

### Beispiel 1:

0,178 ml mol TiCl₄ wurden unter Stickstoffatmosphäre und Rühren bei Raumtemperatur zu einer Lösung von 1,13 g (c-C₇H₁₃)₆Si₆O₇(OH)₄ in 30 ml Toluol gegeben. Zu der entstandenen Reaktionsmischung gab man 0,65 ml Pyridin zu, worauf eine gelbliche Suspension entstand. Diese Suspension wurde 2 Minuten lang auf 50 °C erhitzt. Anschließend ließ man wieder auf Raumtemperatur abkühlen, fügte 0,5 ml Wasser hinzu, rührte einige Zeit, filtrierte von entstandenem Pyridiniumchlorid ab und engte das Filtrat im Vakuum ein. Der erhaltene weißliche Feststoff wurde an der Luft aus einer Mischung aus 20 ml Toluol und 50 ml Acetonitril kristallisiert. Man erhielt 0,80 g mikrokristallines
[(c-C₇H₁₃)₆Si₆O₁₁]₃[TiOH]₄, ¹H-NMR(300 MHz, CDCl₃, 23 °C, SiMe₄): δ = 1,90-1,75 (m, 4H), 1,59-1,35 (m, 8H), 0,91-0,87 (m, 1H).
Ausbeute: 86 %.

## Patentansprüche

1. Verfahren zur Herstellung von Silasesquioxan-Metallkomplexen, **dadurch gekennzeichnet, daß** Halogenide, insbesondere Chloride, C₁-C₂₀-alkyl- oder Aryl-Verbindungen, die kein Betawasserstoff enthalten, z. B. Methyl-, Benzyl-, Neopentyl-, Xylyl-, Mesityl-, Neophil-, Adamantyl-Verbindungen, Silyl, Fluorenyl, Indenyl-, Cyclopentadienyl-Verbindungen, wobei die einzelnen Substituenten durch C₁-C₄-alkyl-, C₂-C₄alkylsilyl, alkoxy-, aryl- oder arylsilyl-Gruppen substituiert sein können,
Oxide, Imide, Amide, Alkoxide (z. B. -OR), wobei R Wasserstoff, C₁-C₂₀-alkyl, insbesondere methyl, ethyl, isopropyl, tert. butyl, aryl, insbesondere benzyl, phenyl, toluyl, naphthyl, xylyl, bedeuten)
oder deren Mischverbindungen z. B. Oxohalogenide, Aryl- oder Alkylhalogenide, Halogenamide oder Alkylalkoxide der Metalle der 4. bis 7. Nebengruppe des PSE
und mindestens ein Silasesquioxan der allgemeinen Formel I
{(R¹SiO_{1,5})ₙ(R²SiO_{1,5})ₘ[(H)ₚ(B)_{q}(O)ᵣ]}
in der
R¹ C₅-C₁₀-cycloalkyl, norbornyl, adamantyl
R² OH
B H, OH, Halogen, alkoxy, SiR³_{y}, wobei R³, C₁-C₄-alkyl, aryl sein kann,
SiMe₂(CH₂)ₛ CH = CH₂, SiMe₂(CH₂)_{S}CH₂CH₂A, SiMe₂ (CH₂)ₛCHACH₃, wobei A für OH, COOH, NH₂, SO₃⁻, COO-, und s für 1 bis 20 steht, y für 2 und 3 steht, vorzugsweise SiR₃y, wobei R³, C₁-C₄-alkyl, aryl sein kann, SiNe₂(CH₂)ₛCH = CH₂, SiMe₂(CH₂)ₛCH₂CH₂A, SiMe₂ (CH₂)ₛCHACH₃, wobei A für OH, COOH, NH₂, SO₃⁻, COO⁻, und s für 1 bis 20 steht, y für 2 und 3 steht und
R¹ und R³ durch Halogen oder OH funktionalisiert sein können,
und
n 6
m 0 und 1
p 0 bis 4
q 0 bis 2
r 0 bis 2
bedeuten,
in einem organischen Lösungsmittel, gegebenenfalls in Gegenwart einer basischen Verbindung, unter Rühren bei -80 °C bis +110 °C suspendiert werden und das Reaktionsprodukt bei Raumtemperatur abgetrennt wird.

2. Verfahren zur Herstellung von Silasesquioxan-Metallkomplexen nach Anspruch 1, **dadurch gekennzeichnet, daß** als Metallverbindung
TiCpCl₃ oder Ti(η⁵-C₅H₃)(SiMe₃-1,3)Cl₃
und mindestens ein Silasesquioxan der allgemeinen Formel I bei 20 bis 50 °C in Toluol suspendiert werden und Pyridin als
basische Verbindung unter Rühren eingetragen und das Reaktionsprodukt abgetrennt wird.

3. Verfahren zur Herstellung von Silasesquioxan-Metallkomplexen nach Anspruch 1, **dadurch gekennzeichnet, daß** als Metallverbindung Zirkonalkyl oder Zirkonalkylaryl und mindestens ein Silasesquioxan der allgemeinen Formel I bei -80 °C bis -20 °C in Toluol suspendiert und unter Rühren auf Raumtemperatur erwärmt werden und das Reaktionsprodukt abgetrennt wird.

4. Verfahren zur Herstellung von Silasesquioxan-Metallkomplexen nach Anspruch 1, **dadurch gekennzeichnet, daß** als Metallverbindung
TiCl₄
und mindestens ein Silasesquioxan der allgemeinen Formel I bei 20 bis 50 °C in Toluol suspendiert werden und Pyridin als basische Verbindung unter Rühren eingetragen wird und das Reaktionsprodukt abgetrennt wird.

5. Verfahren zur Herstellung von silasesquioxan-Metallkomplexen nach Anspruch 1, **dadurch gekennzeichnet, daß** als Metallverbindung VO(OiAlk)₃, insbesondere VO(OiProp)₃ oder VOCl₃ und mindestens ein Silasesquioxan der allgemeinen Formel I in Toluol suspendiert werden und das Reaktionsprodukt abgetrennt wird.

6. Silasesquioxan-Metallkomplexe der allgemeinen Formel II
{(R¹SiO_{1,5})ₙ(R^{2a}SiO_{1,5})ₘ[(B)ₘ(O)ᵣ]}ᵤ(M)ᵥ(Y)_{w}
in der
R¹, B,
n, m, q, r
die Bedeutung gemäß Anspruch 1 haben und
M, Y für die Metallverbindung gemäß Anspruch 1 steht, wobei M für das Metall und Y für die in Anspruch 1 genannten Reste, die an das Metall gebunden sind, steht,
R^{2a} Sauerstoff
u 1 bis 4
v 1 bis 4
w 0 bis 12
bedeuten.

7. Metallkomplexe nach Anspruch 6, umfassend ein oxidisches Metallcluster, **dadurch gekennzeichnet, daß** jedes Metallatom 4- oder 6-fach koordiniert ist und die Komplexe in wäßriger Lösung stabil sind.

8. Metallkomplexe gemäß Anspruch 7, worin das oxidische Metallcluster 4 Titanatome in tetraedrischer Anordnung enthält, wobei jedes Titanatom in erster Koordinationssphäre oktaedrisch von 6 Sauerstoffatomen umgeben ist.

9. Metallkomplexe gemäß Anspruch 8, worin an die jeweils ein Titanatom oktaedrisch umgebenden Sauerstoffatome zusätzlich Atome von Wasserstoff, Kohlenstoff und/oder Silicium gebunden sein können.

10. Metallkomplexe gemäß Anspruch 9, worin mindestens 2 der Titanatome in dem oxidischen Metallcluster über eine Hydroxylgruppe miteinander verbrückt sind.

11. Metallkomplexe gemäß Anspruch 10, worin jeweils 3 der 4 Titanatome die gleiche koordinative Umgebung besitzen und jeweils 3 der 4 Hydroxylgruppen die gleiche koordinative Umgebung besitzen.

12. Metallkomplexe gemäß Anspruch 10, worin die verbleibenden drei freien Koordinationsstellen der 6-fach koordinierten Titanatome durch jeweils ein zu Silasesquioxan-Liganden gehörendes Sauerstoffatom besetzt sind.

13. Metallkomplexe gemäß Anspruch 12, welche drei Silasesquioxan-Liganden der allgemeinen Formel [R₆(Si₆O₇)O₄], worin R für C₅-C₁₀-Cycloalkyl, Adamantyl oder für Norbornyl steht, enthalten.

14. Metallkomplexe gemäß Anspruch 13 der allgemeinen Formel III 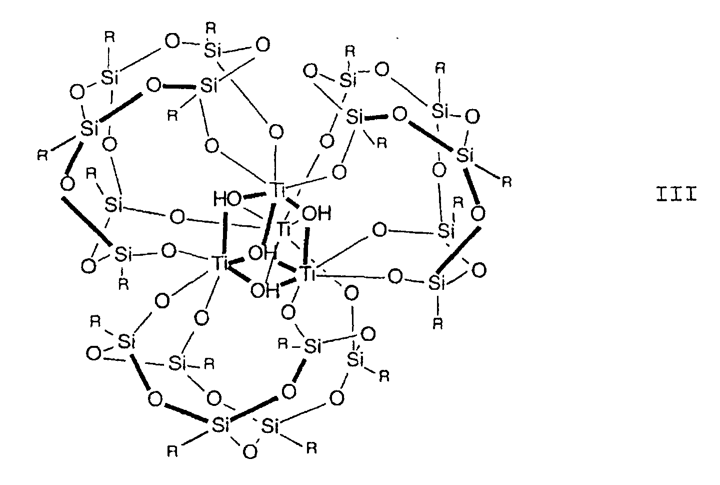 worin R für R¹ steht.

15. Metallkomplexe gemäß Anspruch 14, worin R Cycloheptyl oder Norbornyl bedeutet.

16. Trägerkatalysatoren, umfassend Metallkomplexe gemäß einem der vorstehenden Ansprüche, wobei die Metallkomplexe auf einem üblichen oxidischen Trägermaterial mit einer durchschnittlichen Porenweite von 30 bis 200 Å geträgert sind.

17. Verwendung der Silasesquioxan-Komplexe gemäß einem der vorstehenden Ansprüches als Katalysator zur Oxidation oder Epoxidation von ungesättigten Kohlenwasserstoffen oder Alkoholen.

18. Verwendung gemäß Anspruch 17, wobei die Oxidation in wäßrigem Medium durchgeführt wird.

19. Verwendung gemäß Anspruch 18, wobei als Oxidationsmittel Wasserstoffperoxid eingesetzt wird.

20. Verfahren zur Herstellung einer Epoxid-Verbindung, **dadurch gekennzeichnet, daß** man einen ungesättigten Kohlenwasserstoff oder Alkohol mit einem organischen oder anorganischen Hydroperoxid in Anwesenheit eines gegebenenfalls geträgerten Metallkomplexes gemäß Anspruch 6 umsetzt.

21. Verfahren gemäß Anspruch 20, **dadurch gekennzeichnet, daß** die Reaktion in wäßrigem Medium durchgeführt wird.

22. Verfahren gemäß Anspruch 21, **dadurch gekennzeichnet, daß** H₂O₂ als Oxidationsmittel verwendet wird.

## Claims

1. A process for preparing silasesquioxane-metal complexes, **characterized in that** halides, in particular chlorides, C₁-C₂₀-alkyl or aryl compounds containing no beta-hydrogen, e.g. methyl, benzyl, neopentyl, xylyl, mesityl, neophyl, adamantyl compounds, silyl, fluorenyl, indenyl, cyclopentadienyl compounds, where the individual substituents may be substituted by C₁-C₄-alkyl, C₁-C₄-alkylsilyl, alkoxy, aryl or arylsilyl groups,
oxides, imides, amides, alkoxides (e.g. -OR , where R is hydrogen, C₁-C₂₀-alkyl, in particular methyl, ethyl, isopropyl, tert-butyl, aryl, in particular benzyl, phenyl, tolyl, naphthyl, xylyl)
or their mixed compounds, e.g. oxyhalides, aryl halides or alkyl halides, haloamides or alkyl alkoxides,of the metals of transition groups 4 to 7 of the Periodic Table of the Elements,
and at least one silasesquioxane of the general formula I
{(R¹SiO_{1.5})ₙ(R²SiO_{1.5})ₘ[(H)ₚ(B)_{q}(O)ᵣ]}
where
R¹ is C₅-C₁₀-cycloalkyl, norbornyl, adamantyl,
R² is OH
B is H, OH, halogen, alkoxy, SiR³_{y}, where R³ can be C₁-C₄-alkyl or aryl,
SiMe₂ (CH₂) ₛCH=CH₂, SiMe₂ (CH₂) ₛCH₂CH₂A, SiMe₂ (CH₂)ₛCHACH₃, where A is OH, COOH, NH₂, SO₃⁻, COO⁻, and s is from 1 to 20, y is 2 or 3, preferably SiR³y, where R³ can be C₁-C₄-alkyl or aryl, SiMe₂(CH₂)ₛCH=CH₂, Si-Me₂(CH₂)ₛCH₂CH₂A, SiMe₂(CH₂)ₛCHACH₃, where A is OH, COOH, NH₂, SO₃⁻, COO⁻, and s is from 1 to 20, y is 2 or 3,and
R¹ and R³ may be functionalized by halogen or OH, and
n is 6,
m is 0 or 1,
p is from 0 to 4,
q is from 0 to 2
r is from 0 to 2,
are suspended in an organic solvent, in the presence or absence of a basic compound, with stirring at from -80°C to +110°C and the reaction product is separated off at room temperature.

2. A process for preparing silasesquioxane-metal complexes according to claim 1, **characterized in that**
TiCpCl₃ or Ti(η⁵-C₅H₃) (SiMe₃-1,3)Cl₃
as metal compound and at least one silasesquioxane of the general formula I are suspended in toluene at from 20 to 50°C, and pyridine is introduced as basic compound while stirring,and the reaction product is separated off.

3. A process for preparing silasesquioxane-metal complexes according to claim 1, **characterized in that** a zirconium alkyl or zirconium alkylaryl as metal compound and at least one silasesquioxane of the general formula I are suspended in toluene at from -80°C to -20°C and the suspension is warmed to room temperature while stirring and the reaction product is separated off.

4. A process for preparing silasesquioxane-metal complexes according to claim 1, **characterized in that**
TiCl₄
as metal compound and at least one silasesquioxane of the general formula I are suspended in toluene at from 20 to 50°C and pyridine is introduced as basic compound while stirring and the reaction product is separated off.

5. A process for preparing silasesquioxane-metal complexes according to claim 1, **characterized in that** VO(OiAlk)₃, in particular VO(OiProp)₃, or VOCl₃ as metal compound and at least one silasesquioxane of the general formula I are suspended in toluene and the reaction product is separated off.

6. A silasesquioxane-metal complex of the general formula II
{(R¹SiO_{1.5})ₙ(R^{2a}SiO_{1.5})ₘ[(B)_{q}(O)ᵣ]}ᵤ(M)ᵥ(Y)_{w}
where
R¹, B,
n, m, q, r
are as defined in claim 1 and
M, Y representsthe metal compound according to claim 1, where M is the metal and Y is the radicals specified in claim 1 which are bound to the metal,
R^{2a} is oxygen,
u is from 1 to 4,
v is from 1 to 4,
w is from 0 to 12.

7. A metal complex according to claim 6 comprising an oxidic metal cluster, **characterized in that** each metal atom is tetracoordinated or hexacoordinated and the complex is stable in aqueous solution.

8. A metal complex according to claim 7, wherein the oxidic metal cluster contains 4 titanium atoms in a tetrahedral arrangement and each titanium atom is surrounded octahedrally by 6 oxygen atoms in the first coordination sphere.

9. A metal complex according to claim 8, wherein hydrogen, carbon and/or silicon atoms may additionally be bound to the oxygen atoms which octahedrally surround each titanium atom.

10. A metal complex according to claim 9, wherein at least two of the titanium atoms in the oxidic metal cluster are bridged via a hydroxyl group.

11. A metal complex according to claim 10, wherein 3 of the 4 titanium atoms have the same coordination environment and 3 of the 4 hydroxyl groups have the same coordination environment.

12. A metal complex according to claim 10, wherein the remaining three free coordination sites of the hexacoordinated titanium atoms are each occupied by an oxygen atom of a silasesquioxane ligand.

13. A metal complex according to claim 12 containing three silasesquioxane ligands of the general formula [R₆(Si₆O₇)O₄],
where R is C₅-C₁₀-cycloalkyl, adamantyl or norbornyl.

14. A metal complex according to claim 13 of the general formula III 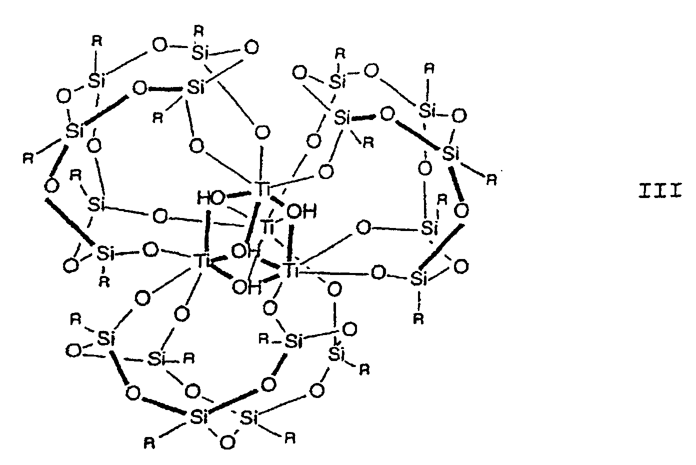 where R is as defined for R¹.

15. A metal complex according to claim 14, wherein R is cyclopheptyl or norbornyl.

16. A supported catalyst comprising a metal complex according to any one of the preceding claims 6-15, wherein the metal complex is supported on a customary oxidic support material having an average pore size of from 30 to 200 Å.

17. The use of a silasesquioxane complex according to any one of the preceding claims 6-15 as catalyst for the oxidation or epoxidation of unsaturated hydrocarbons or alcohols.

18. The use according to claim 17, wherein the oxidation is carried out in an aqueous medium.

19. The use according to claim 18, wherein hydrogen peroxide is used as oxidant.

20. A process for preparing an epoxide compound, **characterized in that** an unsaturated hydrocarbon or alcohol is reacted with an organic or inorganic hydroperoxide in the presence of a supported or unsupported metal complex according to claim 6.

21. A process according to claim 20, **characterized in that** the reaction is carried out in an aqueous medium.

22. A process according to claim 21, **characterized in that** H₂O₂ is used as oxidant.

## Revendications

1. Procédé de production de complexes silasesquioxane - métal,
**caractérisé en ce que**
des halogénures, en particulier des chlorures, des composés alkylés en C₁-C₂₀, ou des composés aryles qui ne contiennent aucun hydrogène en bêta, par exemple les composés méthyle, benzyle, néopentyle, xylyle, mesityle, néophile, et adamantyle, des composés silyle, fluorényle, indenyle et cyclopentadiènyle, dont les substituants individuels peuvent être substitués par des groupes alkyle en C₁-C₄, des alkyl en C₁-C₄ - silyles , des groupes alkoxy, aryl ou arylsilyl,
des oxydes, des imides, des amides, des alkoxydes (par exemple -OR dans lequel R signifie de l'hydrogène, un alkyle en C₁-C₂₀, en particulier méthyle éthyle, isopropyle, terbulyle, aryle - en particulier benzyle, to-luyle, phényle, naphtyle, xylyle), ou leurs composés mixtes par exemples des oxohalogénures, des halogénures d'aryle ou d'alkyle, des halogénamides, ou des alkoxyles d'alkyle des métaux du 4^{ème} au 7^{ème} sous-groupe de la classification périodique des éléments,
et au moins un silasesquioxane de formule générale I
(R¹SiO_{1,5})ₙ(R²SᵢO_{1,5})m[(H)ₚ(B)_{q}(O)ᵣ]
dans laquelle
R¹ signifie un cycloalkyle en C₅-C₁₀, un norbornyle, un adamantyle,
R² signifie un OH
B signifie H, OH, halogène, alkoxy, Si R³y pour lequel R³ peut être un alkyle en C₁-C₄ ou un aryle,
Si Me₂(CH₂)₅CH = CH₂, Si Me₂(CH₂) = CH₂CH₂A,
Si Me₂(CH₂) CHACH₃, pour lesquels A représente OH, COOH, NH₂, SO₃-, COO⁻ et s représente de 1 à 20, y représente 2 ou 3, de préférence Si R₃Y dans lequel R³ peut être un alkyle en C₁-C₄ ou un aryle,
Si Me₂(CH₂)ₛCH = CH₂, SiMe₂(CH₂)ₛ CH₂CH₂A, Si Me₂(CH₂)ₛ CH A CH3 dans laquelle A représente OH, COOH NH₂, SO₃⁻, COO- et s représente de 1 à 20, y représente 2 et 3 et
R¹ et R³ peuvent être fonctionnalisés par un halogène ou par un OH et
n signifie 6
m signifie 0 et 1
p signifie 0 à 4
q signifie 0 à 2
r signifie 0 à 2
sont mis en suspension
dans un solvant organique, le cas échéant en présence d'un composé basique sous agitation à de -80°C à + 110°C et le produit réactionnel est séparé à température ambiante.

2. °Procédé de production de complexes silasesquioxane - métal selon la revendication 1,
**caractérisé en ce qu'**
on met en suspension comme composé métallique :
TiCₚCl₃ ou Ti (η₅-C₅H₃) (SiMe₃-1,3)Cl₃
et au moins un silasesquioxane de formule générale 1
à 20 à 50°C dans le toluène
et on introduit la pyridine comme composé basique sous agitation et on sépare le produit réactionnel.

3. Procédé de production de complexe silasesquioxane - métal selon la revendication 1,
**caractérisé en ce qu'**
on met en suspension comme composé métallique un zirconalkyle ou un zirconalkylaryle et au moins un silasesquioxane de formule générale I à de -80°C à -20°C dans le toluène, et on réchauffe sous agitation à température ambiante et on sépare le produit réactionnel.

4. Procédé de production de complexes silasesquioxane-métal selon la revendication 1,
**caractérisé en ce qu'**
on met en suspension comme composé métallique
TiCl₄
et au moins un silasesquioxane de formule générale 1 à de 20 à 50°C dans le toluène et on introduit la pyridine comme composant basique sous agitation et on sépare le produit réactionnel.

5. Procédé de production de complexes silasesquioxane-métal selon la revendication 1,
**caractérisé en ce qu'**
on met en suspension comme composé métallique VO (Oᵢ Alk)₃, en particulier VO (OᵢProp)₃ ou VOCl₃ et au moins un silasesquioxane de formule générale 1 dans le toluène et on sépare le produit réactionnel.

6. Complexes silasesquioxane-métal de formule générale (II),
{(R¹SiO_{1,5})ₙ(R^{2a}SiO_{1,5})ₘ[(B)_{q}(O)ᵣ]}ᵤ(M)ᵥ(Y)_{w}
dans laquelle R¹, B, n, m, q et r ont les significations conformément à la revendication 1, M, et Y représentent le composé métallique conformément à la revendication 1, M étant le métal et Y étant les restes cités à la revendication 1 qui sont liés au métal
R^{2a} signifie de l'oxygène
u signifie de 1 à 4
v signifie de 1 à 4
w signifie de 0 à 12.

7. Complexes métalliques selon la revendication 6,
comprenant un cluster métallique oxydique
**caractérisé en ce que**
chaque atome de métal est coordonné 4 ou 6 fois et les complexes sont stables en solution aqueuse.

8. Complexes métalliques conformément à la revendication 7,
dans lesquels le cluster métallique oxydique renferme 4 atomes de titane dans une disposition tétrahédrique, dans laquelle chaque atome de titane est entouré dans une première sphère de coordination, d'une manière octaédrique par six atomes d'oxygène.

9. Complexes métalliques conformément à la revendication 8,
dans lesquels sur les atomes d'oxygène entourant à chaque fois un atome de titane d'une manière octaédrique, en supplément des atomes d'hydrogène, de carbone, et/ou de silicium peuvent être liés.

10. Complexes métalliques conformément à la revendication 9,
dans lesquels au moins deux des atomes de titane dans le cluster métallique oxydique sont reliés par un pont l'un avec l'autre par l'intermédiaire d'un groupe hydroxyle.

11. Complexes métalliques conformément à la revendication 10,
dans lesquels à chaque fois trois des quatre atomes de titane possèdent le même environnement coordinatif et à chaque fois 3 des 4 groupes hydroxyle possèdent le même environnement coordinatif.

12. Complexes métalliques conformément à la revendication 10,
dans lesquels les trois emplacements de coordination libres qui demeurent des atomes de titane 6 fois coordinés sont occupés par à chaque fois un atome d'oxygène qui appartient aux ligands de silasesquioxane.

13. Complexes métalliques conformément à la revendication 12, qui renferment trois ligands de silasesquioxane de formule générale,
[R⁶(Si₆O₇)O₄],
dans laquelle R représente un cycloalkyl en C₅-C₁₀, un adamantyle ou un norbornyle.

14. Complexes métalliques conformément à la revendication 13, de formule générale III, 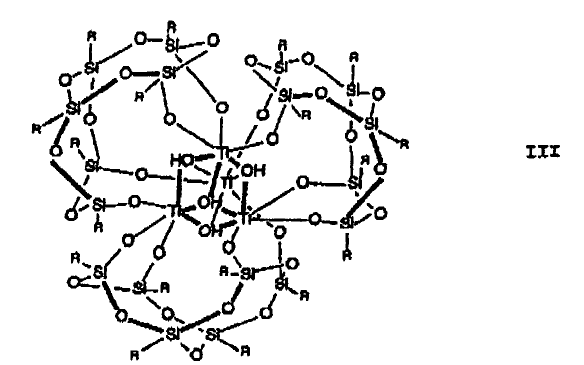 dans laquelle R représente R¹.

15. Complexes métalliques conformément à la revendication 14,
dans lesquels R signifie cycloheptyle ou norbornyle.

16. Catalyseurs sur support, comprenant les complexes métalliques conformément à l'une des revendications précédentes, dans lesquels les complexes métalliques sont mis sur support sur un matériau de support oxydique usuel ayant une largeur de pores moyenne de 30 à 200 Å.

17. Utilisation des complexes de silasesquioxane conformément à l'une des revendications précédentes,
comme catalyseur d'oxydation ou d'époxydation d'hydrocarbures non saturés ou d'alcools.

18. Utilisation conformément à la revendication 17,
dans laquelle on effectue l'oxydation en milieu aqueux.

19. Utilisation conformément à la revendication 18,
dans laquelle on met en oeuvre comme agent d'oxydation, du peroxyde d'hydrogène.

20. Procédé de production d'un composé époxidique,
**caractérisé en ce qu'**
on met à réagir un hydrocarbure non saturé ou un alcool avec un hydroperoxyde organique ou mineral, une présence d'un complexe métallique éventuellement sur support conformément à la revendication 6.

21. Procédé conformément à la revendication 20,
**caractérisé en ce que**
la réaction est effectuée en milieu aqueux

22. Procédé conformément à la revendication 21,
**caractérisé en ce qu'**
on utilise H₂O₂ comme agent d'oxydation.
